# EUROPÄISCHE PATENTSCHRIFT

(11) **EP 1 243 370 B1**
(45) Veröffentlichungstag und Bekanntmachung des Hinweises auf die Patenterteilung: **10.01.2007**
(21) Anmeldenummer: 02004653.8
(22) Anmeldetag: 28.02.2002
(51) Int. Cl.: B23D 65/00, B23D 61/02

(54) **Verfahren zur Herstellung eines chirurgischen Sägeblatts**
Method for making a surgical saw blade
Méthode de production d'une lame de scie chirurgicale

(30) Priorität: 14.03.2001 DE 10112286
(43) Veröffentlichungstag der Anmeldung: 25.09.2002
(73) Patentinhaber: Gebr. Brasseler GmbH & Co. KG, D-32657 Lemgo (DE)
(72) Erfinder: Küllmer, Michael, 32657 Lemgo (DE)
(74) Vertreter: Weber, Joachim

(56) Entgegenhaltungen:
- CH-A- 676 198
- DE-A- 3 833 735
- US-A- 4 584 999
- US-A- 5 569 257

## Beschreibung

Die Erfindung bezieht sich auf ein Verfahren zur Herstellung eines chirurgischen Sägeblatts mit einem Einspannbereich und einem mit einer Verzahnung versehenen Arbeitsbereich.

Chirurgische Sägeblätter der beschriebenen Art sind aus dem Stand der Technik in unterschiedlichen Ausgestaltungsformen bekannt. Sie werden an Antriebseinheiten montiert, durch welche sie in eine oszillierende, hin- und hergehende Bewegung versetzt werden. Dabei wird der kreisbogenförmige Verzahnungsbereich entsprechend in Bewegung gesetzt, so dass ein Schnittvorgang möglich ist. Der Säge- oder Schnittvorgang kann entweder durch manuelles Führen erfolgen, alternativ hierzu ist es auch möglich, das chirurgische Sägeblatt mittels einer Schablone zu verwenden, um eine präzise Schnittführung sicherzustellen.

Sägeblätter der beschriebenen Art werden aus dünnem, hochfestem Stahl gefertigt, wobei die Dicken sich in Größenordnungen zwischen beispielsweise 0,7 und 0,2 mm bewegen.

Die CH-A-676 198 beschreibt ein Verfahren zur Herstellung eines Sägeblattes für eine Oszillationssäge. Dabei werden mehrere derartige Sägeblätter aus einem kreisrunden, scheibenförmigen Rohling gefertigt. Das Fertigen kann entweder durch Schneiden mittels eines Laserstrahls oder durch Ätzen erfolgen.

Der Erfindung liegt die Aufgabe zugrunde, ein Verfahren zur Herstellung eines chirurgischen Sägeblatts zu schaffen, welches bei einfacher Ausgestaltung und einfacher Anwendbarkeit sowohl kostengünstig durchführbar ist als auch die Herstellung von hochpräzisen Sägeblättern ermöglicht.

Erfindungsgemäß wird die Aufgabe durch die Merkmale des Hauptanspruchs gelöst, die Unteransprüche zeigen weitere vorteilhafte Ausgestaltungen der Erfindung.

Erfindungsgemäß ist somit vorgesehen, dass der Einspannbereich und der Arbeitsbereich mittels eines Ätzverfahrens hergestellt werden und dass die Verzahnung nachfolgend mittels eines Laser-Schneidverfahrens geschnitten wird.

Das erfindungsgemäße Sägeblatt zeichnet sich durch eine Reihe erheblicher Vorteile aus.

Durch die Verwendung eines Form-Ätzverfahrens ist es möglich, die Außenkontur der Sägeblätter schnell und vor allem kostengünstig zu erzeugen und dabei die erforderliche Präzision zu realiseren. Bei der Erzeugung der Verzahnung ist ein Ätzverfahren nicht vorteilhaft, da sich bei einem Ätzverfahren stets abgerundete oder zumindest geringfügig verrundete Kanten ergeben. Dies ist für scharfe Schneidkanten unbrauchbar. Mittels des erfindungsgemäßen Laser-Schneidverfahrens kann somit die Verzahnung selbst in präziser Weise so hergestellt werden, dass sich scharfe Schneidkanten ergeben. Ein Nacharbeiten oder Schleifen der Verzahnung ist hierbei nicht erforderlich.

Bei der Erfindung ist vorgesehen, dass die Verzahnung mittels des Ätzverfahrens mit Toleranz vorgefertigt und mit dem LaserSchneidverfahren fertig gestellt wird. Der durch das LaserSchneidverfahren abzutragende Materialbereich kann somit deutlich minimiert werden.

Üblicherweise sind in den chirurgischen Sägeblättern im Einspannbereich Ausnehmungen ausgearbeitet, um eine Befestigung an einer Antriebseinheit zu ermöglichen. Diese Ausnehmungen können, ebenso wie Ausnehmungen im Arbeitsbereich, erfindungsgemäß mittels des Ätzverfahrens besonders einfach und kostengünstig hergestellt werden. Insbesondere ist dies in gleichen Arbeitsschritten möglich wie die Herstellung der Außenkontur.

Ein weiterer Vorteil der erfindungsgemäßen Vorgehensweise liegt darin, dass mittels des Ätzverfahrens unterschiedliche Dickenbereiche des Einspannbereichs und/oder des Arbeitsbereiches erzeugt werden können. So ist es beispielsweise möglich, die Dicke des Einspannbereichs unverändert - verglichen mit dem Ausgangsmaterial - beizubehalten, während der Arbeitsbereich eine verringerte Dicke aufweisen kann. Besonders vorteilhaft ist es, wenn die Verzahnung eine gegenüber dem Arbeitsbereich vergrößerte Dicke hat, insbesondere im Bereich der Zahnspitzen der Verzahnung. Hierdurch wird die Schneidleistung erhöht. Zum anderen erhöht sich die mechanische Festigkeit der Verzahnung selbst.

Das Sägeblatt ist erfindungsgemäß bevorzugterweise aus einem chirurgischen Stahl gefertigt, wobei insbesondere martensitische Stähle oder austensitische Stähle mit einer Festigkeit > 1000 N/mm² zur Anwendung kommen können, die auf die erforderliche Härte härtbar sind oder eine entsprechende Grundhärte aufweisen.

Im Folgenden wird die Erfindung anhand zweier Ausführungsbeispiele in Verbindung mit der Zeichnung beschrieben. Dabei zeigt:
- Fig. 1: eine Seitenansicht eines ersten Ausführungsbeispiels eines erfindungsgemäßen chirurgischen Sägeblatts,
- Fig. 2: eine Seitenansicht des in Fig. 1 gezeigten Sägeblatts,
- Fig. 3: eine Ansicht analog Fig. 3 eines weiteren Ausführungsbeispiels des erfindungsgemäßen Sägeblatts und
- Fig. 4: eine Seitenansicht des in Fig. 3 gezeigten Sägeblatts.

Wie sich aus den Figuren ergibt, weist das Sägeblatt jeweils einen Einspannbereich 1 auf, der mit zumindest einer Ausnehmung 5 versehen ist, die zur Einspannung und Lagerung in einer Antriebseinheit dient. Da die Antriebseinheiten und die Lagerungsmöglichkeiten dem Stand der Technik entsprechen, kann auf eine detaillierte Beschreibung an dieser Stelle verzichtet werden.

Anschließend an den Einspannbereich ist ein Arbeitsbereich 3 ausgebildet, dessen vorderes Ende mit einer Verzahnung 2 versehen ist. Die Verzahnung 2 ist bogenförmig angeordnet, wobei der Mittelpunkt des Bogens dem Schwenkmittelpunkt des in der Antriebseinheit montierten Sägeblatts gleich ist.

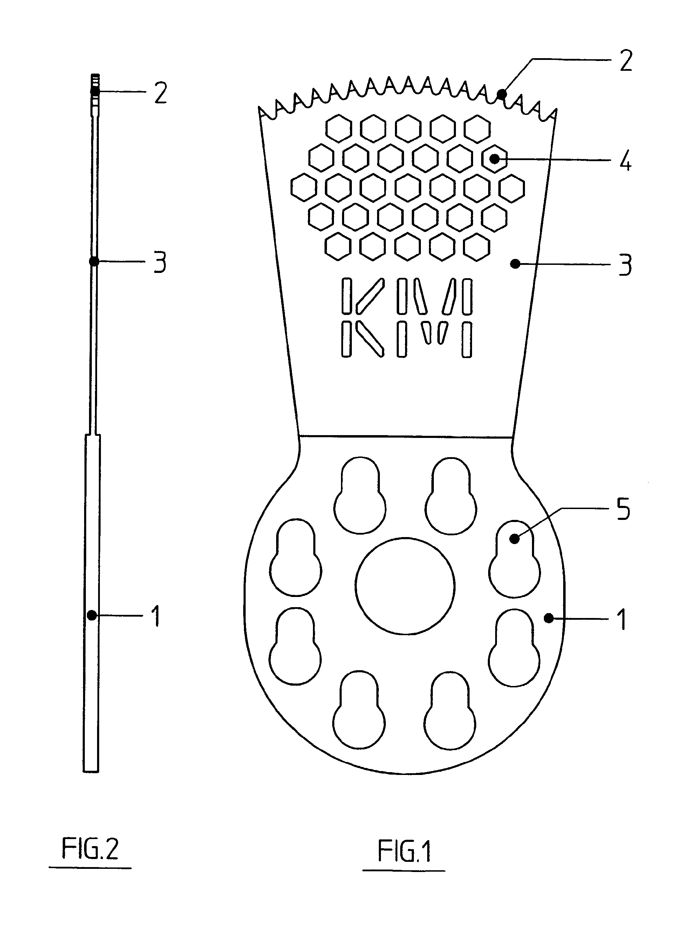

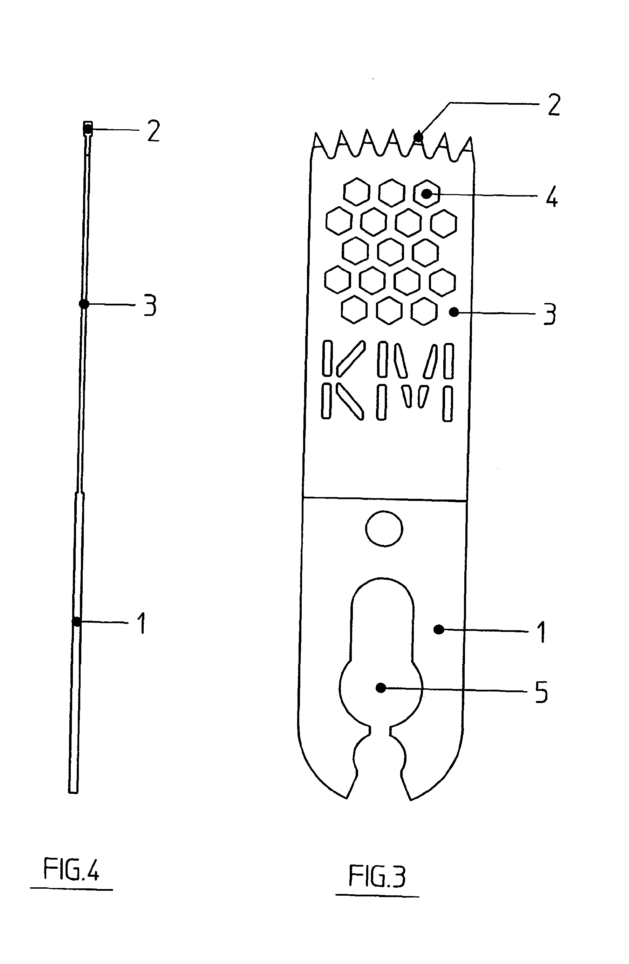

Die Seitenansichten der Fig. 2 und 4 zeigen jeweils, dass die Sägeblätter unterschiedliche Dicken haben können. Bei dem Ausführungsbeispiel der Fig. 1 und 2 kann beispielsweise der Einspannbereich eine Dicke von 0,5 mm aufweisen, während der Arbeitsbereich mit einer Dicke von 0,27 mm versehen ist. Die Verzahnung 2 wiederum kann eine Dicke von 0,35 mm haben. Insgesamt ist das Sägeblatt beispielsweise 35 mm lang, es weist an seinem Einspannbereich einen Durchmesser von 16 mm auf, während die Verzahnung eine Breite von 15 mm hat.

Wie sich weiterhin aus den Fig. 1 und 2 ergibt, können bevorzugterweise im Bereich des Arbeitsbereichs 3 zusätzliche Ausnehmungen 4 vorgesehen sein, um während des Arbeitsvorganges den Blick auf den Sägebereich zu ermöglichen.

Das in den Fig. 3 und 4 gezeigte Ausführungsbeispiel zeigt ein schmales, längliches Sägeblatt. Gleiche Teile sind mit gleichen Bezugsziffern wie bei dem vorangegangenen Ausführungsbeispiel bezeichnet. Das Sägeblatt kann beispielsweise eine Gesamtlänge von 34 mm und eine Breite von 8 mm haben. Die Dicke des Einspannbereichs kann beispielsweise 0,38 mm betragen. Die Verzahnung kann die gleiche Dicke haben, während der Arbeitsbereich 3 eine Dicke von lediglich 0,2 mm hat.

Die Verringerung der Dicken der Arbeitsbereiche 3 ist mittels des erfindungsgemäßen Form-Ätzverfahrens herstellbar. Die Erfindung ist nicht auf die gezeigten Ausführungsbeispiele beschränkt, vielmehr ergeben sich im Rahmen der Erfindung vielfältige Abwandlungs- und Modifikationsmöglichkeiten.

## Patentansprüche

1. Verfahren zur Herstellung eines chirurgischen Sägeblatts mit einem Einspannbereich (1) und einem mit einer Verzahnung (2) versehenen Arbeitsbereich (3), **dadurch gekennzeichnet, dass** der Einspannbereich (1) und der Arbeitsbereich (3) mittels eines Ätzverfahrens hergestellt werden und dass die Verzahnung (2) mittels des Ätzverfahrens mit Aufmaß vorgefertigt und nachfolgend mittels eines Laser-Schneidverfahrens fertiggeschnitten wird.

2. Verfahren nach Anspruch 1, **dadurch gekennzeichnet, dass** die Verzahnung (2) mittel des Laser-Schneidverfahrens gebrauchsfertig erzeugt wird.

3. Verfahren nach einem der Ansprüche 1 oder 2, **dadurch gekennzeichnet, dass** Ausnehmungen (4, 5) in dem Einspannbereich (1) und/oder dem Arbeitsbereich (3) mittels des Ätzverfahrens hergestellt werden.

4. Verfahren nach einem der Ansprüche 1 bis 3, **dadurch gekennzeichnet, dass** mittels des Ätzverfahrens unterschiedliche Dickenbereiche des Einspannbereichs (1) und/oder des Arbeitsbereichs (3) erzeugt werden.

5. Verfahren nach Anspruch 4, **dadurch gekennzeichnet, dass** der Einspannbereich (1) mit einer größeren Dicke als der Arbeitsbereich (3) ausgebildet wird und dass die Verzahnung (2) eine gegenüber dem Arbeitsbereich (3) vergrößerte Dicke hat.

6. Verfahren nach einem der Ansprüche 1 bis 5, **dadurch gekennzeichnet, dass** das Sägeblatt aus chirurgischen Stahl gefertigt ist.

## Claims

1. Method for making a surgical saw blade having a clamping region (1) and a working region (3) provided with a tooth system (2), **characterized in that** the clamping region (1) and the working region (3) are produced by means of an etching method, and **in that** the tooth system (2) is prefabricated with an allowance by means of the etching method and is subsequently cut to size by means of a laser cutting method.

2. Method according to Claim 1, **characterized in that** the tooth system (2) is produced in ready-to-use form by means of the laser cutting method.

3. Method according to either of Claims 1 and 2, **characterized in that** recesses (4, 5) are produced in the clamping region (1) and/or in the working region (3) by means of the etching method.

4. Method according to any of Claims 1 to 3,
**characterized in that** different thickness regions of the clamping region (1) and/or of the working region (3) are produced by means of the etching method.

5. Method according to Claim 4, **characterized in that** the clamping region (1) is formed with a larger thickness than the working region (3), and **in that** the tooth system (2) has a greater thickness than the working region (3).

6. Method according to any of Claims 1 to 5,
**characterized in that** the saw- blade is produced from surgical steel.

## Revendications

1. Procédé de fabrication d'une lame de scie chirurgicale, comportant une zone de fixation (1) et une zone de travail (3) munie d'une denture (2), **caractérisé en ce que** la zone de fixation (1) et la zone de travail (3) sont réalisées au moyen d'un procédé d'attaque chimique et **en ce que** la denture (2) est réalisée avec un surdimensionnement initial au moyen d'un procédé d'attaque acide et est découpée ensuite à la forme finie par un procédé de découpe par laser.

2. Procédé selon la revendication 1, **caractérisé en ce que** la denture (2) est réalisée prête à l'emploi au moyen du procédé de découpe par laser.

3. Procédé selon la revendication 1 ou 2, **caractérisé en ce que** des évidements (4, 5) sont réalisés dans la zone de fixation (1) et/ou dans la zone de travail (3) au moyen du procédé d'attaque chimique.

4. Procédé selon l'une quelconque des revendications 1 à 3, **caractérisé en ce que** des zones d'épaisseur différente sont réalisées au moyen du procédé d'attaque chimique dans la zone de fixation (1) et/ou dans la zone de travail (3).

5. Procédé selon la revendication 4, **caractérisé en ce que** la zone de fixation (1) est réalisée avec une plus grande épaisseur que la zone de travail (3) et **en ce que** la denture (2) a une épaisseur supérieure à celle de la zone de travail (3).

6. Procédé selon l'une quelconque des revendications 1 à 5, **caractérisé en ce que** la lame de scie est réalisée en acier chirurgical.
